(19) Europäisches Patentamt
European Patent Office
Office européen des brevets

(11) Veröffentlichungsnummer: **0 237 608 B1**

(12) **EUROPÄISCHE PATENTSCHRIFT**

(45) Veröffentlichungstag der Patentschrift: **29.01.92**

(51) Int. Cl.[5]: **C07D 405/12**, A61K 31/505

(21) Anmeldenummer: **86103925.3**

(22) Anmeldetag: **21.03.86**

Die Akte enthält technische Angaben, die nach dem Eingang der Anmeldung eingereicht wurden und die nicht in dieser Patentschrift enthalten sind.

(54) **Kristalline, wasserfreie Sigma -Form von 2-[4-(2-Furoyl-(2-piperazin)-1-yl]-4-amino-6,7-dimethoxychinazolinhydrochlorid und Verfahren zu ihrer Herstellung.**

(43) Veröffentlichungstag der Anmeldung:
**23.09.87 Patentblatt 87/39**

(45) Bekanntmachung des Hinweises auf die Patenterteilung:
**29.01.92 Patentblatt 92/05**

(84) Benannte Vertragsstaaten:
**AT BE CH DE FR GB IT LI LU NL SE**

(56) Entgegenhaltungen:
**LU-A- 85 616**
**US-A- 4 092 315**

(73) Patentinhaber: **HEUMANN PHARMA GMBH & CO**
**Heideloffstrasse 18 - 28**
**W-8500 Nürnberg 1(DE)**

(72) Erfinder: **Schickaneder, Helmut, Dr. Dipl. Chem.**
**Moosäcker 25**
**W-8501 Eckental(DE)**
Erfinder: **Grafe, Ingomar, Dr. Dipl.Chem.**
**Auerbacher Strasse 18**
**W-8500 Nürnberg 30(DE)**
Erfinder: **Ahrens, Kurt Henning, Dr.**
**Praterstrasse 9**
**W-8500 Nürnberg(DE)**

(74) Vertreter: **Kraus, Walter, Dr. et al**
**Patentanwälte Kraus, Weisert & Partner**
**Thomas-Wimmer-Ring 15**
**W-8000 München 22(DE)**

EP 0 237 608 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft eine neue wasserfreie, stabile, kristalline Form von 2-[4-(2-Furoyl)-piperazin-1-yl]-4-amino-6,7-dimethoxychinazolinhydrochlorid (Prazosin-Hydrochlorid. Prazosin-Hydrochlorid ist ein bekanntes antihypertensiv wirksames Mittel der folgenden Formel:

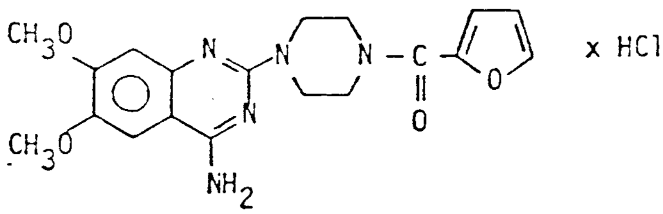

Dieser Wirkstoff wurde erstmals in der US-PS 3 511 836 beschrieben.

Danach sind verschiedene polymorphe Formen des Prazosin-Hydrochlorids bekannt geworden. So werden in der DE-AS 27 08 192 die α-Form, die β-Form, die γ-Form, ein Polyhydrat/Dihydrat, das Monohydrat, die δ-Form und ein Methanolat beschrieben. Nach den Angaben dieser Druckschrift werden den einzelnen Formen bestimmte charakteristische IR-Banden und charakteristische Spitzen im Beugangsdiagramm zugeordnet. Gemäß der DE-AS 27 08 192 soll die α-Form von Prazosin-Hydrochlorid aufgrund ihrer geringen Hygroskopizität und Beständigkeit bedeutende Vorteile in der Handhabung, Lagerung und Formulierung besitzen.

Eine weitere kristalline Form von Prazosin-Hydrochlorid wird in der DE-OS 34 29 415 beschrieben. Nach den Angaben dieser Druckschrift soll die dort als δ-Form bezeichnete Substanz relativ unhygroskopisch sein. Aufgrund der in dieser Durckschrift angegebenen charakteristischen Banden im Infrarotspektrum ist anzunehmen, daß die δ-Form der DE-OS 34 29 415 mit der δ-Form von Prazosin-Hydrochlorid, die in der DE-AS 27 08 192 beschrieben wird, identisch ist.

Der vorliegenden Erfindung liegt nun die Aufgabe zugrunde, eine neue, bislang nocht nicht bekannte, außergewöhnlich stabile und stets reproduzierbare kristalline Form von Prazosin-Hydrochlorid zur Verfügung zu stellen.

Diese Aufgabe wird erfindungsgemäß durch die kristalline wasserfreie σ-Form von 2-[4-(2-Furoyl)-piperazin-1-yl]-4-amino-6,7-dimethoxychinazolinhydrochlorid, erhältlich durch stufenweise Umsetzung von 2-[4-(2-Furoyl)-piperazin-1-yl]-4-amino-6,7-dimethoxychinazolin mit Eisessig und wäßriger Ammoniumchloridlösung und anschließendes Trocknen, Umsetzung der erhaltenen getrockneten σ-Form von 2-[4-(2-Furoyl)-piperazin-1-yl]-4-amino-6,7-dimethoxychinazolinhydrochiorid-hydrat in Methanol und Trocknen der resultierenden 2-[4-(2-Furoyl)-piperazin-1-yl]-4-amino-6,7-dimethoxychinazolinhydrochlorid-methanolat-σ-Form unter Entfernung des Kristallmethanolanteils, gelöst.

Die erfindungsgemäße σ-Form hat Absorptionsbanden bei den in der nachstehenden Tabelle angegebenen Wellenlängen bzw. Frequenzen (in Kaliumbromid).

TABELLE

| Banden | | |
|---|---|---|
| µm | $cm^{-1}$ | |
| 2.90 | 3445 | Singulett (scharf) |
| 9.90 | 1010 | Duplett |
| 13.03 | 768 | Singulett (scharf) |

Wie oben ausgeführt, ist die erfindungsgemäße σ-Form von 2-[4-(2-Furoyl)-piperazin-1-yl]-4-amino-6,7-dimethoxychinazolinhydrochlorid dadurch erhältlich, daß man

a) 2-[4-(2-Furoyl)-piperazin-1-yl]-4-amino-6,7-dimethoxychinazolin (Prazosin-Base) der Formel I

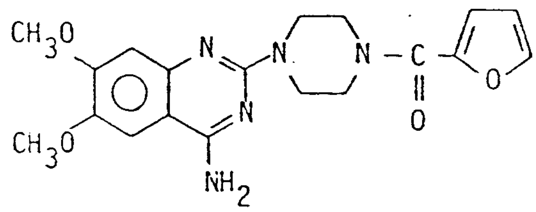

(I)

stufenweise in einer wäßrigen Säure-Base-Reaktion umsetzt;

b) die erhaltene, sehr gut getrocknete Prazosin-Hydrochlorid-hydrat-σ-Form der Formel II

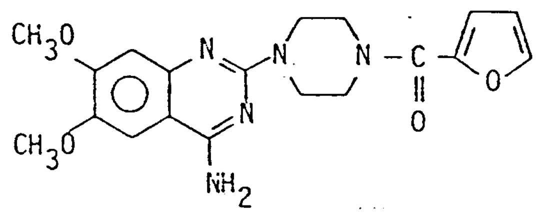

x HCl x $H_2O$ (II)

in Methanol umsetzt; und

c) anschließend die erhaltene Prazosin-Hydrochloridmethanolat-σ-Form der Formel III

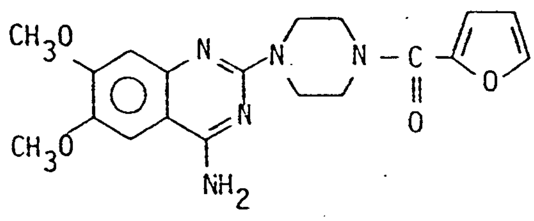

x HCl x $CH_3OH$ (III)

trocknet.

Im folgenden wird dieses Verfahren anhand seiner einzelnen Stufen näher beschrieben.

I. Stufe (a)

In der ersten Stufe setzt man 2-[4-(2-Furoyl)-piperazin-1-yl]-4-amino-6,7-dimethoxychinazolin (Prazosin-Base) der Formel I

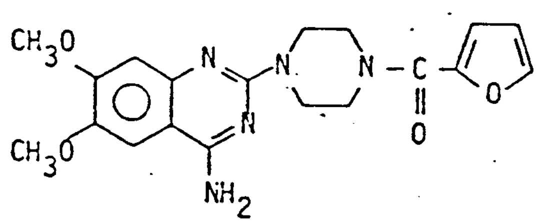

(I)

in einer Reaktionslösung bei erhöhten Temperaturen, z.B. bei 30 °C bis 100 °C, vorzugsweise bei 75 °C, um, wobei die Prazosin-Base und Eisessig in unterschiedlichen Molverhältnissen, bevorzugt im Molverhältnis 1 zu 2, eingesetzt werden. Die Reaktionslösung besteht vorzugsweise aus Methylglykol, Wasser und Eisessig, doch läßt sich die Umsetzung auch mit anderen Polyglykolen, z.B. Diethylenglykol, Wasser und

schwachen organischen Säuren, wie z.B. Ameisensäure, Propionsäure, Buttersäure, Glykolsäure, Glycerinsäure, Isobuttersäure, n-Valeriansäure oder Capronsäure, durchführen, wobei jedoch die Umsetzung in Methylglykol, Wasser und Eisessig bevorzugt wird.

Anschließend wird das Reaktionsgemisch bei erhöhten Temperaturen, z.B. 30 bis 100 °C, vorzugsweise bei 80 bis 90 °C, mit einer äquimolaren oder vorzugsweise überschüssigen molaren (z.B. 30%), bezogen auf Prazosin-Base, wäßrigen Ammoniumchloridlösung umgesetzt. Die Reaktionsdauer beträgt 10 bis 60 Minuten, z.B. ca. 30 Minuten. Das erhaltene Produkt wird sehr gut getrocknet, was beispielsweise bei 100 °C im Vakuum erfolgen kann.

II. Stufe (b)

In der zweiten Stufe reagiert die sehr gut getrocknete Prazosin-hydrochlorid-hydrat-σ-Form in Methanol innerhalb von 5 bis 60 Minuten, bevorzugt in 30 Minuten, bei 20 °C bis Rückflußtemperatur des Lösungsmittels, vorzugsweise bei Rückflußtemperatur, zur Prazosin-hydrochloridmethanolat-σ-Form ab.

III. Stufe (c)

Die Umwandlung der Prazosin-hydrochlorid-methanolat-σ-Form in die erfindungsgemäße wasserfreie Prazosin-hydrochlorid-σ-Form erfolgt durch Entfernen des Kristallmethanolanteils.

Bevorzugt wird dabei die Trocknung der Prazosin-hydrochlorid-methanolat-σ-Form im Vakuum bei ca. 90 bis 120 °C, vorzugsweise bei 105 bis 110 °C, durchgeführt. Die Trocknung dauert ca. 4 bis 20 Stunden, vorzugsweise 15 Stunden.

Die erfindungsgemäße neue Form von Prazosin-Hydrochlorid besitzt gegenüber den bislang bekannten polymorphen Formen dieses Wirkstoffs eine in überraschender Weise erhöhte Stabilität. Dies hat sich aufgrund von Stabilitätstests ergeben, in denen die erfindungsgemäße σ-Form des Prazosin-Hydrochlorids bei unterschiedlichen Lagerungsbedingungen getestet wurde. Hierbei wurden die Proben beispielsweise 12 Wochen bei 45 °C, 55 °C und 75 °C gelagert. Danach wurden sie anhand der IR-Spektren und mit Hilfe der Hochdruckflüssigkeitschromatographie (Gehaltsbestimmung) überprüft. Auch bei 3wöchiger Lagerung im Tageslicht wurden bei den Proben keine visuellen Veränderungen beobachtet.

Die Untersuchung verschiedener Proben in wäßriger Suspension bei pH-Werten von 1 bis 10 innerhalb von 6 Wochen ohne und mit Tageslichteinfluß sowie bei 45 °C, 55 °C und 75 °C (12 Wochen-Dauer) ergab keine visuellen Änderungen. Die Parameter der quantitativen Bestimmungen blieben ebenfalls unverändert.

Weiterhin haben entsprechende Vergleichsversuche ergeben, daß die erfindungsgemäße neue σ-Form von Prazosin-Hydrochlorid hinsichtlich der Stabilität der aus der DE-AS 27 08 192 bekannten α-Form von Prazosin-Hydrochlorid überlegen ist.

Die erfindungsgemäße neue σ-Form des Prazosin-Hydrochlorids ist nicht hygroskopisch. Dies bedeutet, daß bei der erfindungsgemäßen neuen Form der Wassergehalt nach längerer Lagerzeit nicht über 1,5% beträgt.

Prazosin-Hydrochlorid in σ-Form gemäß der Erfindung mit bis zu, aber nicht wesentlich mehr als etwa 1,5% Wasser, wird hierin als wasserfrei angesehen.

Die Erfindung wird anhand der Figur und der folgenden Beispiele erläutert. Die Figur stellt das Infrarotspektrum der erfindungsgemäßen σ-Form von Prazosin-Hydrochlorid dar.

**Beispiel 1**

a) Prazosin-hydrochlorid-hydrat-σ-Form

In einer 50-l-Reaktionsapparatur werden 2.4 kg (6.53 mol) Prazosin-Base in 9 l Methylglykol, 8.5 l Wasser und 7 l (12.2 mol) Eisessig suspendiert und auf ca. 75 °C erhitzt, bis Lösung eingetreten ist. Zur Lösung gibt man je 100 g Tonsil und 100 g Aktivkohle und überführt die Reaktionslösung bei ca. 90 bis 100 °C durch eine Klärfiltration über eine beheizte Filterstrecke in einen 100-l-Reaktionskessel.

Zu dem Filtrat gibt man noch heiß (ca. 80 bis 90 °C) eine Lösung von 450 g (8.4 mol) Ammoniumchlorid - gelöst in ca. 15 l Wasser - innerhalb 10 Minuten zu, wobei das Prazosin-Hydrochlorid fällt. Man rührt noch ca. 15 Minuten und kühlt dabei auf 20 °C ab. Das Produkt wird abgesaugt, mit ca. 2.5 l Wasser und dann mit 2.5 l Methanol nachgewaschen. Das möglichst gut abgepreßte Produkt wird bei 100 °C im Vakuum sehr gut getrocknet.

Ausbeute an Prazosin-hydrochlorid-hydrat-σ-Form getrocknet: 1.9 kg

b) Prazosin-hydrochlorid-methanolat-σ-Form

Man trägt die oben erhaltene getrocknete Hydratform (1.9 kg) in 7.6 l Methanol und erhitzt ca. 30 Minuten auf Rückflußtemperatur. Anschließend saugt man ab und wäscht mit 1.9 l Methanol nach.
Nach dem Trocknen an der Luft erhält man 2.0 kg Prazosin-hydrochlorid-methanolat-σ-Form.
$C_{19}H_{21}N_5O_4$ x HCl x $CH_3OH$ (451.5)
Ber.: C 53.15 H 5.80 N 15.50 Cl 7.85
Gef.: C 53.10 H 5.71 N 15.38 Cl 7.81

Schmelzpunkt (bestimmt durch DTA): 265° C (Zers.), wobei zwischen 110 bis 160° C Eliminierung von Methanol eintritt.
Anmerkung: Die Methanolatform des α-Prazosin schmilzt bzw. zersetzt sich bei 275° C.

**Beispiel 2**

Prazosin-Hydrochlorid-σ-Form

2.0 kg Prazosin-hydrochlorid-methanolat-σ-Form werden bei 105 bis 110° C im Vakuum (10 mbar) 15 Stunden getrocknet.
Ausbeute: 1.7 kg (61%, bezogen auf Prazosin-Base)
Schmelzpunkt (bestimmt durch DTA): 265° C (Zers.)
IR: ($KBr^{-1}$)γ = 3445 cm $^{-1}$, 1010 $cm^{-1}$, 768 $cm^{-1}$ (charakteristische Banden)
$C_{19}H_{21}N_5O_4$ x HCL (419,5)
Ber.: C 54.34 H 5.28 N 16.68 Cl 8.44
Gef.: C 54.31 H 5.22 N 16.60 Cl 8.32
Anmerkung: α-Prazosin schmilzt bzw. zersetzt sich bei 275° C.

**Patentansprüche**
**Patentansprüche für folgende Vertragsstaaten : BE, CH, DE, FR, GB, IT, LI, LU, NL, SE**

**1.** Kristalline, wasserfreie σ-Form von 2-[4-(2-Furoyl)-piperazin-1-yl]-4-amino-6,7-dimethoxychinazolinhydrochlorid, erhältlich durch stufenweise Umsetzung von 2-[4-(2-Furoyl)-piperazin-1-yl]-4-amino-6,7-dimethoxychinazolin mit Eisessig und wäßriger Ammoniumchloridlösung und anschließendes Trocknen, Umsetzung der erhaltenen getrockneten σ-Form von 2-[4-(2-Furoyl)-piperazin-1-yl]-4-amino-6,7-dimethoxychinazolinhydrochlorid-hydrat in Methanol und Trocknen der resultierenden 2-[4-(2-Furoyl)-piperazin-1-yl]-4-amino-6,7-dimethoxychinazolinhydrochlorid-methanolat-σ-Form unter Entfernung des Kristallmethanolanteils.

**2.** Arzneimittel, dadurch **gekennzeichnet,** daß es neben üblichen Hilfs- und Trägerstoffen die kristalline, wasserfreie σ-Form von 2-[4-(2-Furoyl)-piperazin-1-yl]-4-amino-6,7-dimethoxychinazolinhydrochlorid nach Anspruch 1 enthält.

**Patentanspruch für folgenden Vertragsstaat : AT**

**1.** Verfahren zur Herstellung der σ-Form von 2-[4-(2-Furoyl)-piperazin-1-yl]-4-amino-6,7-dimethoxychinazolinhydrochlorid, dadurch **gekennzeichnet,** daß man 2-[4-(2-Furoyl)-piperazin-1-yl]-4-amino-6,7-dimethoxychinazolin stufenweise mit Eisessig und wäßriger Ammoniumchloridlösung umsetzt und anschließend trocknet, die erhaltene getrocknete σ-Form von 2-[4-(2-Furoyl)-piperazin-1-yl]-4-amino-6,7-dimethoxychinazolinhydrochlorid-hydrat in Methanol umsetzt und daß man die resultierende 2-[4-(2-Furoyl)-piperazin-1-yl]-4-amino-6,7-dimethoxy-chinazolinhydrochlorid-methanolat-σ-Form unter Entfernung des Kristallmethanolanteils trocknet.

**Claims**
**Claims for the following Contracting States : BE, CH, DE, FR, GB, IT, LI, LU, NL, SE**

**1.** Crystalline, anhydrous σ-form of 2-[4-(2-furoyl)piperazin-1-yl]-4-amino-6,7-dimethoxyquinazoline hydrochloride obtainable by the stepwise reaction of 2-[4-(2-furoyl)-piperazin-1-yl]-4-amino-6,7-dimethoxyquinazoline with glacial acetic acid and aqueous ammonium chloride solution followed by drying,

reaction of the resulting dried σ-form of 2-[4-(2-furoyl)-piperazin-1-yl]-4-amino-6,7-dimethoxy-quinazoline hydrochloride hydrate in methanol and drying of the resulting 2-[4-(2-furoyl)-piperazin-1-yl]-4-amino-6,7-dimethoxy-quinazoline hydrochloride methanolate-σ form with removal of the crystalline methanol component.

**2.** Medicament, characterised in that in addition to containing the usual auxiliary substances and carriers it contains the crystalline, anhydrous σ-form of 2-[4-(2-furoyl)-piperazin-1-yl]-4-amino-6,7-dimethoxy-quinazoline hydrochloride according to claim 1.

**Claim for the following Contracting State : AT**

**1.** A process for the preparation of the σ-form of 2-[4-(2-furoyl)-piperazin-1-yl]-4-amino-6,7-dimethoxy-quinazoline hydrochloride, characterised in that 2-[4-(2-furoyl)-piperazin-l-yl]-4-amino-6,7-dimethoxyquinazoline is reacted stepwise with glacial acetic acid and aqueous ammonium chloride solution and then dried, the resulting dried σ-form of 2-[4-(2-furoyl)-piperazin-1-yl]-4-amino-6,7-dimethoxyquinazoline hydrochloride is reacted in methanol and the resulting 2-[4-(2-furoyl)-piperazin-1-yl]-4-amino-6,7-dimethoxy-quinazoline hydrochloride methanolate σ-form is dried with removal of the crystalline methanol component.

**Revendications**

**Revendications pour les Etats contractants suivants : BE, CH, DE, FR, GB, IT, LI, LU, NL, SE**

**1.** Forme σ cristallisée anhydre du chlorhydrate de 2-[4-(2-furoyl)-pipérazine-1-yl]-4-amino-6,7-diméthoxyquinazoline, accessible par réaction par étapes de la 2-[4-(2-furoyl)-pipérazine-1-yl]-4-amino-6,7-diméthoxyquinazoline avec l'acide acétique et une solution aqueuse de chlorure d'ammonium suivie de séchage, réaction de la forme σ de l'hydrate de chlorhydrate de 2-[4-(2-furoyl)-pipérazine-1-yl]-4-amino-6,7-diméthoxyquinazoline séchée obtenue dans le méthanol et séchage de la forme σ du méthanolate de chlorhydrate de 2-[4-(2-furoyl)-pipérazine-1-yl]-4-amino-6,7-diméthoxyquinazoline résultante avec élimination du méthanol de cristallisation.

**2.** Médicament, caractérisé en ce qu'il contient en plus des substances auxiliaires et supports habituels la forme σ cristalliséeanhydre du chlorhydrate de 2-[4-(2-furoyl)-pipérazine-1-yl]-4-amino-6,7-diméthoxyquinazoline selon la revendication 1.

**Revendication pour l'Etat contractant suivant : AT**

**1.** Procédé pour la fabrication de la forme σ du chlorhydrate de 2-[4-(2-furoyl)-pipérazine-1-yl]-4-amino-6,7-diméthoxyquinazoline, caractérisé en ce que l'on fait réagir par étapes la 2-[4-(2-furoyl)-pipérazine-1-yl]-4-amino-6,7-diméthoxyquinazoline avec l'acide acétique et une solution aqueuse de chlorure d'ammonium et ensuite on sèche, on fait réagir ta forme σ de l'hydrate de chlorhydrate de 2-[4-(2-furoyl)-pipérazine-1-yl]-4-amino-6,7-diméthoxyquinazoline séchée obtenue dans le méthanol et on sèche la forme σ du méthanotate de chlorhydrate de 2-[4-(2-furoyl)-pipérazine-1-yl]-4-amino-6,7-diméthoxyquinazoline résultante avec élimination du méthanol de cristallisation.

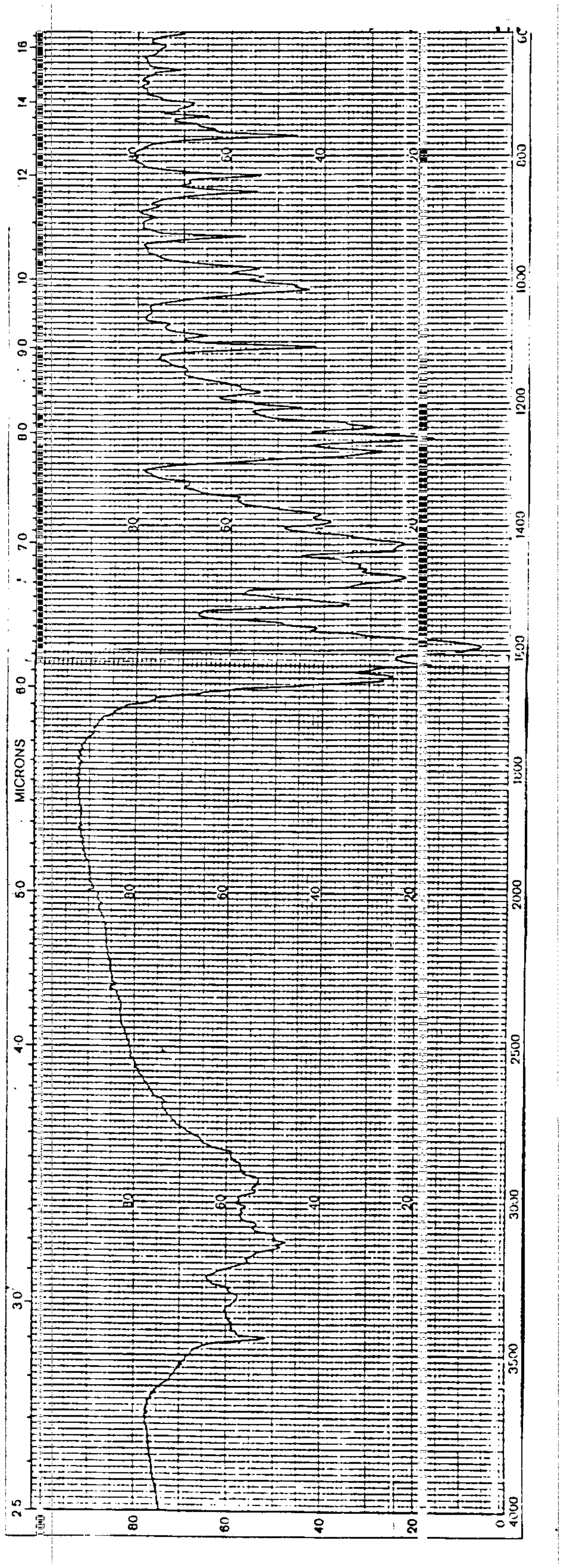
MICRONS